# EUROPEAN PATENT APPLICATION

(11) **EP 4 768 073 A1**
(43) Date of publication of application: **01.07.2026**
(21) Application number: 24865811.4
(22) Date of filing: 10.09.2024
(51) Int. Cl.: A61N 5/06, A61N 5/00

(54) **NEAR INFRARED RAY TREATMENT DEVICE**

(30) Priority: 15.09.2023 KR 20230122834
(71) Applicant: Shin, Seong Bok, Seoul 07776 (KR)
(72) Inventor: Shin, Seong Bok, Seoul 07776 (KR)
(74) Representative: Nederlandsch Octrooibureau
(86) International application number: PCT/KR2024/013718
(87) International publication number: WO 2025/058372

(57) **Abstract**

The present disclosure relates to a near infrared treatment device. The near infrared treatment device includes: a substrate; a plurality of near infrared light emitting diode (LED) chips mounted on one surface of the substrate and emitting near infrared light; and a lens module including at least one lens arranged adjacent to the plurality of near infrared LED chips and concentrating the near infrared light output from the plurality of near infrared LED chips and irradiating the near infrared light onto a treatment site, thereby improving a treatment effect.

## Description

### Technical Field

The present disclosure relates to a near infrared treatment device, and more particularly, to a near infrared treatment device that concentrates near infrared light output from a plurality of near infrared light emitting diode (LED) chips by using a lens module and irradiates the near infrared light onto a treatment site, thereby improving a treatment effect of the near infrared treatment device and improving durability of the near infrared treatment device.

### Background Art

In general, an infrared treatment device is a treatment device that transfers heat to skin or muscle tissue by using infrared light. The infrared treatment device emits thermal energy of infrared radiation and transfers the thermal energy to a deep layer of the skin, and thus is used for various medical and health-related purposes such as pain relief, muscle relaxation, and improvement of blood circulation.

Infrared light is a general term for a band of 700 nm to 1 mm among bands having a wavelength longer than a visible light wavelength band (400 nm to 700 nm). Infrared light is a representative form of radiant energy that directly transfers energy from a high-temperature object to a low-temperature object without a medium. Thus, infrared light is widely used for medical purposes to transfer energy to human skin or deep tissues.

Most infrared treatment devices are configured to irradiate infrared light onto a treatment site by using an infrared lamp, such as a halogen lamp, in order to maximize a treatment effect of infrared radiation.

For example, various structures of an infrared treatment device using an infrared lamp are disclosed in Korean Patent Laid-Open Publication No. 10-2019-0002225 (Lamp Coupling Structure for Infrared Treatment Device) (published on January 8, 2019) and Korean Patent Registration No. 10-2161564 (Infrared Lamp Device for Thermal Treatment Device) (published on October 5, 2020).

However, the conventional infrared treatment device has a problems in that the distribution of infrared light itself irradiated from an infrared lamp is not uniform. Furthermore, the infrared light is irradiated to a wide area of a treatment site by using the infrared lamp. Accordingly, the infrared light is dispersed, which reduces a treatment effect. Due to this, the conventional infrared treatment device has a problem in that the overall size increases and the structure becomes complicated because a large lens has to be provided in order to irradiate a narrow area of a treatment site.

In addition, the conventional infrared treatment device has an inconvenience of having to be used close to a treatment site in order to overcome a reduction in treatment effect caused by dispersion of infrared light. Furthermore, the conventional infrared treatment device has a problem in that durability is significantly reduced due to heat generation from the infrared lamp.

On the other hand, recently, a near infrared treatment device using near infrared light has also been developed. For example, various structures of a near infrared treatment device using near infrared light are disclosed in Korean Utility Model Registration No. 20-0290062 (Personal Thermal Treatment Device Using Near Infrared Light) (published on September 26, 2002) and Korean Patent Laid-Open Publication No. 10-2022-0130935 (Near Infrared LED Heater Module for Light Treatment) (September 27, 2022).

However, the conventional near infrared treatment device has a problem in that, similar to an infrared lamp, near infrared light is irradiated over a wide area of a treatment site so that the near infrared light is dispersed, and thus, a treatment effect is reduced.

Therefore, there is a need for a near infrared treatment device capable of improving a treatment effect of the near infrared treatment device and improving durability of the near infrared treatment device.

### Disclosure of Invention

### Technical Problem

The present disclosure has been made in an effort to improve the above-described problems, and the problem to be solved by the present disclosure is to provide a near infrared treatment device that concentrates near infrared light output from a plurality of near infrared light emitting diode (LED) chips by using a lens module and irradiates the near infrared light onto a treatment site, thereby improving a treatment effect of the near infrared treatment device.

The objects of the present disclosure are not limited to those described above, and other objects that are not disclosed herein will be clearly understood from the following description by those of ordinary skill in the art.

### Solution to Problem

To achieve the objects described above, a near infrared treatment device according to an embodiment of the present disclosure includes: a substrate; a plurality of near infrared light emitting diode (LED) chips mounted on one surface of the substrate and emitting near infrared light; and a lens module including at least one lens arranged adjacent to the plurality of near infrared LED chips and concentrating the near infrared light output from the plurality of near infrared LED chips and irradiating the near infrared light onto a treatment site.

In this case, the plurality of near infrared LED chips generate near infrared light having a wavelength band of any one of 830 nm, 850 nm, and 940 nm.

In addition, the plurality of near infrared LED chips may be mounted on the substrate in a chip-on-board (COB) method.

In addition, the lens module includes: a first lens arranged above the plurality of near infrared LED chips and collecting the near infrared light output from the plurality of near infrared LED chips; and a second lens arranged above the first lens and concentrating the near infrared light having passed through the first lens on the treatment site in a form of a spot.

In addition, the first lens uses a hemispherical convex lens arranged to surround the plurality of near infrared LED chips and protruding in a hemispherical shape to collect the near infrared light output from the plurality of near infrared LED chips, and the second lens uses a flat block lens that concentrates the near infrared light having passed through the first lens.

On the other hand, the near infrared treatment device further includes a cooling module arranged adjacent to the substrate and cooling heat generated from the plurality of near infrared LED chips.

Specific details of other embodiments are included in the detailed description and drawings.

### Advantageous Effects of Invention

A near infrared treatment device according to an embodiment of the present disclosure may concentrate near infrared light output from a plurality of near infrared light emitting diode (LED) chips by using a lens module and irradiates the near infrared light onto a treatment site, thereby improving a treatment effect of the near infrared treatment device.

In addition, the near infrared treatment device according to an embodiment of the present disclosure includes a cooling module that cools heat generated from the plurality of near infrared LED chips, thereby preventing the near infrared treatment device from being damaged due to heat and improving the durability of the near infrared treatment device.

The effects of the present disclosure are not limited to those described above, and other effects that are not described herein will be clearly understood from the description of the claims by those of ordinary skill in the art.

### Brief Description of Drawings

FIG. 1 is a perspective view schematically illustrating a structure of a near infrared treatment device according to an embodiment of the present disclosure.
FIG. 2 is a longitudinal cross-sectional view schematically illustrating the structure of the near infrared treatment device according to an embodiment of the present disclosure.
FIG. 3 is a diagram schematically illustrating a structure of a plurality of light emitting diode (LED) chips constituting the near infrared treatment device according to an embodiment of the present disclosure.
FIG. 4 is a diagram schematically illustrating a structure of a lens module constituting the near infrared treatment device according to an embodiment of the present disclosure.
FIG. 5 is a diagram schematically illustrating a structure of a cooling module constituting the near infrared treatment device according to an embodiment of the present disclosure.

### Best Mode for Carrying out the Invention

Hereinafter, preferred embodiments of the present disclosure will be described in detail with reference to the accompanying drawings, so that those of ordinary skill in the art can easily carry out the present disclosure.

In describing the embodiments, descriptions of technologies that are well known in the technical field to which the present disclosure belongs and are not directly related to the present disclosure will be omitted. By omitting unnecessary description, the present disclosure may be described more clearly without obscuring the gist of the present disclosure.

For the same reason, some elements in the accompanying drawings are exaggerated, omitted, or schematically illustrated. In addition, the size of each element does not entirely reflect the actual size. The same reference numerals are assigned to the same or corresponding elements in the drawings.

In addition, it will be understood that the expressions and terms as used herein with respect to device or element orientation (e.g., "front," "back," "up," "down," "top," "bottom," "left," "right," "lateral," etc.) are only used to simplify the description of the present disclosure and do not necessarily indicate or imply that the relevant device or element should have a particular direction.

Hereinafter, the present disclosure will be described with reference to drawings for explaining a near infrared treatment device according to an embodiment of the present disclosure.

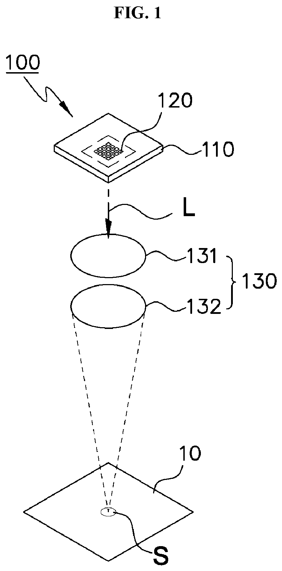

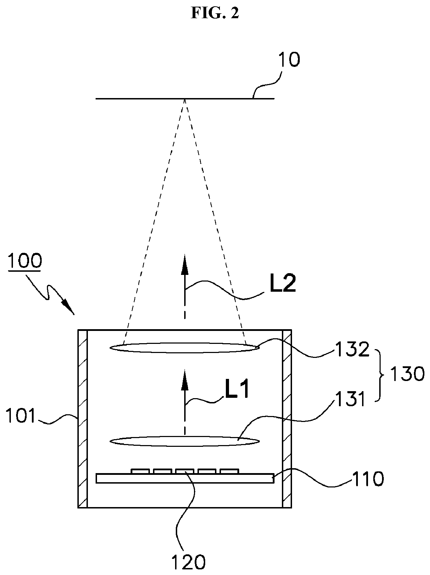

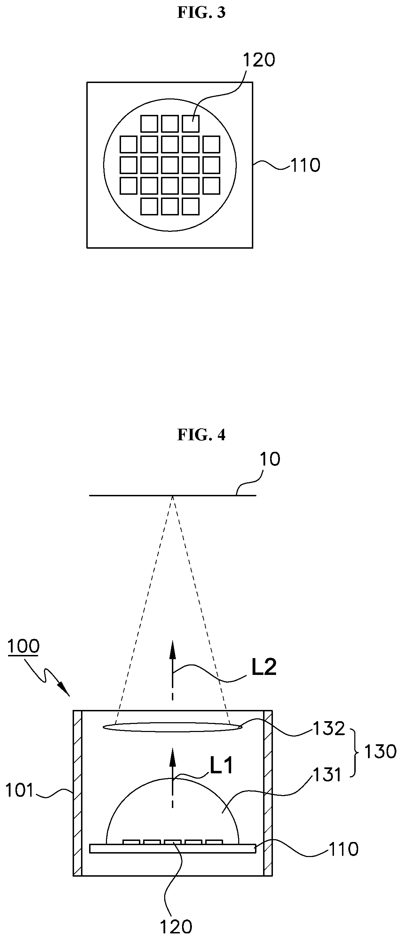

FIG. 1 is a perspective view schematically illustrating a structure of a near infrared treatment device according to an embodiment of the present disclosure, FIG. 2 is a longitudinal cross-sectional view schematically illustrating the structure of the near infrared treatment device according to an embodiment of the present disclosure, FIG. 3 is a diagram schematically illustrating a structure of a plurality of light emitting diode (LED) chips constituting the near infrared treatment device according to an embodiment of the present disclosure, and FIG. 4 is a diagram schematically illustrating a structure of a lens module constituting the near infrared treatment device according to an embodiment of the present disclosure.

As illustrated in FIGS. 1 and 2, a near infrared treatment device 100 according to an embodiment of the present disclosure may be configured to include a substrate 100, a plurality of near infrared LED chips 120, and a lens module 130.

The substrate 100 may be installed inside a housing 101 of the near infrared treatment device 100, and the plurality of near infrared LED chips 120 or the like may be mounted on one surface of the substrate 100 (in the example of FIG. 2, an upper surface of the substrate 100). A printed circuit board, a flexible printed circuit board (FPCB), or the like may be used as the substrate 100.

The plurality of near infrared LED chips 120 may be mounted on one surface of the substrate 100 (in the example of FIG. 2, the upper surface of the substrate 100) and may output near infrared light L having a wavelength band of about 780 nm to about 3,000 nm in order to treat a treatment site.

The near infrared light L may be mainly used for treatment of wounds, lacerations, scars, and inflammation. When light emitted from sunlight or a heating source is dispersed into a spectrum, the near infrared light L is located outside a red spectrum and has a shortest wavelength. Since the near infrared light L has a photoelectric effect and a fluorescent effect as well as a thermal effect, the near infrared light L is used for disinfection, sterilization, and treatment of joints and muscles. Furthermore, the near infrared light L promotes rapid pain relief by generating nitric oxide (NO).

Preferably, the near infrared LED chips 120 constituting the near infrared treatment device 100 according to an embodiment of the present disclosure may generate the near infrared light L having a wavelength band of 830 nm, 850 nm, or 940 nm.

Preferably, as illustrated in FIG. 3, the plurality of near infrared LED chips 120 may be mounted on the substrate 100 in a chip-on-board (COB) method. The COB method is one of the integrated circuit technologies used for manufacturing electronic products and circuits. In the COB method, the near infrared LED chip 120 is directly attached to a printed circuit board, an FPCB, or the like, and thus, additional packaging or case is not required. Accordingly, the overall size may be miniaturized, the manufacturing costs may be reduced, and electrical performance may be improved. In addition, the COB method may have high reliability because the COB method is resistant to shock or vibration.

The lens module 130 may include at least one lens that is arranged at a position adjacent to the plurality of near infrared LED chips 120 and concentrates the near infrared light L output from the plurality of near infrared LED chips 120 and irradiates the near infrared light onto a treatment site.

Preferably, as illustrated in FIG. 4, the lens module 130 may be configured to include a first lens 131 and a second lens 132.

The first lens 131 may be arranged above the plurality of near infrared LED chips 120 and may collect near infrared light L1 output from the plurality of near infrared LED chips 120, and the second lens 132 may be arranged above the first lens 131 and may concentrate near infrared light L2 having passed through the first lens 131 onto the treatment site in the form of a spot (S in FIG. 1).

That is, since the lens module 130 may irradiate the near infrared light L output from the plurality of near infrared LED chips 120 onto the treatment site in the form of a spot, a reduction in treatment effect due to dispersion of the near infrared light L may be prevented. Since the near infrared light L is not dispersed, a sufficient distance from the treatment site may be secured. At this time, when the distance from the treatment site is 1 m, the near infrared light L emitted by the lens module 130 may be irradiated in the form of a spot having a radius of about 200 mm.

Preferably, as illustrated in FIG. 4, the first lens 131 may use a hemispherical convex lens arranged to surround the plurality of near infrared LED chips 120 and protruding in a hemispherical shape to collect the near infrared light L1 output from the plurality of near infrared LED chips 120. In addition, the second lens 132 may use a flat block lens that concentrates the near infrared light L2 having passed through the first lens 131.

As such, the near infrared treatment device 100 according to an embodiment of the present disclosure may concentrate the near infrared light L output from the plurality of near infrared LED chips 120 by using the lens module 130 and irradiate the near infrared light L onto the treatment site, thereby improving a treatment effect of the near infrared treatment device 100.

On the other hand, the near infrared treatment device 100 according to an embodiment of the present disclosure may further include a cooling module 140 that cools heat generated from the plurality of near infrared LED chips 120.

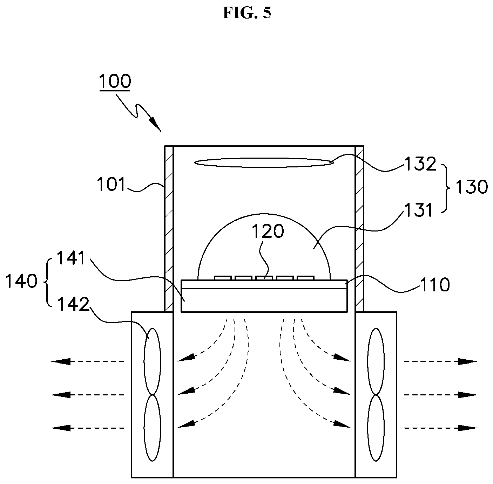

FIG. 5 is a diagram schematically illustrating the structure of the cooling module constituting the near infrared treatment device according to an embodiment of the present disclosure.

As illustrated in FIG. 5, the cooling module 140 may be arranged adjacent to the substrate 100 and may cool heat generated from the plurality of near infrared LED chips 120.

Preferably, the cooling module 140 may have an air-cooling structure that discharges heat generated from the plurality of near infrared LED chips 120 to the outside. That is, as illustrated in FIG. 5, the cooling module 140 may include a heat dissipation plate 141 installed on one surface of the substrate 100 (in the example of FIG. 5, the lower surface of the substrate 100) and dissipating heat generated from the plurality of near infrared LED chips 120, and a cooling fan 142 installed at a position adjacent to the heat dissipation plate 141 and discharging heat transferred from the heat dissipation plate 141 to the outside.

On the other hand, although FIG. 5 illustrates an example in which two cooling fans 142 are installed at both lower ends of one heat dissipation plate 141 arranged on the lower surface of the substrate 110, the present disclosure is not limited thereto, and the number and arrangement of the heat dissipation plates 141 and the cooling fans 142 may be variously changed by those of ordinary skill in the art.

Although not illustrated, the cooling module 140 may include, in addition to the heat dissipation plate 141 and the cooling fan 142, a heat transfer member (not shown) arranged between the substrate 100 and the heat dissipation plate 141 and having high thermal conductivity in order to improve the transfer efficiency of heat generated from the plurality of near infrared LED chips 120 and the substrate 100.

Accordingly, the near infrared treatment device 100 according to an embodiment of the present disclosure includes the cooling module 140 that cools heat generated from the plurality of near infrared LED chips 120, thereby preventing the near infrared treatment device 100 from being damaged due to heat and improving the durability of the near infrared treatment device 100.

Meanwhile, the present specification and drawings disclose preferred embodiments of the present disclosure. Although specific terms are used, these terms are used only in a general sense to easily explain the technical concept of the present disclosure and help understanding of the present disclosure and are not intended to limit the scope of the present disclosure. In addition to the embodiments disclosed herein, it will be apparent to those of ordinary skill in the art that other modifications based on the technical idea of the present disclosure are possible.

### Industrial Applicability

The present disclosure relates to a near infrared treatment device, and more particularly, may be applied to a technical field related to a near infrared treatment device that irradiates near infrared light onto a treatment site, thereby improving a treatment effect.

## Claims

1. A near infrared treatment device comprising:
a substrate;
a plurality of near infrared light emitting diode (LED) chips mounted on one surface of the substrate and emitting near infrared light; and
a lens module comprising at least one lens arranged adjacent to the plurality of near infrared LED chips and concentrating the near infrared light output from the plurality of near infrared LED chips and irradiating the near infrared light onto a treatment site.

2. The near infrared treatment device of claim 1, wherein the plurality of near infrared LED chips generate near infrared light having a wavelength band of any one of 830 nm, 850 nm, and 940 nm.

3. The near infrared treatment device of claim 1, wherein the plurality of near infrared LED chips are mounted on the substrate in a chip-on-board (COB) method.

4. The near infrared treatment device of claim 1, wherein the lens module comprises:
a first lens arranged above the plurality of near infrared LED chips and collecting the near infrared light output from the plurality of near infrared LED chips; and
a second lens arranged above the first lens and concentrating the near infrared light having passed through the first lens on the treatment site in a form of a spot.

5. The near infrared treatment device of claim 4, wherein the first lens uses
a hemispherical convex lens arranged to surround the plurality of near infrared LED chips and protruding in a hemispherical shape to collect the near infrared light output from the plurality of near infrared LED chips, and
the second lens uses a flat block lens that concentrates the near infrared light having passed through the first lens.

6. The near infrared treatment device of claim 1, further comprising a cooling module arranged adjacent to the substrate and cooling heat generated from the plurality of near infrared LED chips.
